# EUROPEAN PATENT APPLICATION

(11) **EP 1 064 944 A1**
(43) Date of publication of application: **03.01.2001**
(21) Application number: 99250207.0
(22) Date of filing: 25.06.1999
(51) Int. Cl.: A61K 31/55

(54) **Protein kinase N inhibitor comprising Fasudil**

(71) Applicant: SCHERING AKTIENGESELLSCHAFT, 13353 Berlin (DE)
(72) Inventor: Ohashi, Yasuhiro, Osaka, 534-0024 (JP); Konno, Yasuhiko, Suita City, Osaka 564-0043 (JP); Miwa, Naoto, Takatsuki City, Osaka 569-1031 (JP)

(57) **Abstract**

Disclosed is (1) a protein kinase N inhibitor comprising hexahydro-1-(5-isoquinolinesulfonyl)-1H-1,4-diazepine or its salts, (2) a dual inhibitor of protein kinase N and p160ROCK comprising hexahydro-1-(5-isoquinolinesulfonyl)-1H-1,4-diazepine or its salts, (3) a pharmaceutical composition for controlling carcinomatous peritonitis derived from intraabdominal tumor comprising hexahydro-1-(5-isoquinolinesulfonyl)-1H-1,4-diazepine or its salts, (4) a pharmaceutical composition for controlling carcinomatous pleuritis derived from intrathoracic tumor comprising hexahydro-1-(5-isoquinolinesulfonyl)-1H-1,4-diazepine or its salts and (5) a pharmaceutical composition for controlling cancer invasion comprising hexahydro-1-(5-isoquinolinesulfonyl)-1H-1,4-diazepine or its salts.

## Description

### TECHNICAL FIELD

This invention is directed to protein kinase N inhibitor and/or a dual inhibitor of protein kinase N and a Rho-associated, coiled-coil containing protein kinase (p160ROCK) comprising hexahydro-1-(5-isoquinolinesulfonyl)-1H-1,4-diazepine or its salts.

### BACKGROUND OF THE INVENTION

Rho (Ras homologue) is a low molecular weight GTP binding protein, which was discovered by Pascal Madaule et.al. in 1985. It is known that Rho protein is related to cancer invasion (FEBS Lett., 372, 25-28 (1995)). Several downstream kinases of Rho have been discovered and reported so far, including, (1) protein kinase N [hereinafter called as "PKN"; identified by molecular cloning from human hippocampus cDNA library (Biochem. Biophys. Res. Commun. 199, N0.2, 897-904 (1994))], (2) p160ROCK (a Rho-associated, coiled-coil containing protein kinase) [hereinafter called as "ROCK"; disclosed in EMBO J. 15, 1885-1893 (1996)], (3) Citron (FEBS Lett. 377, 243-248 (1995)) and (4) Rho kinase (EMBO J. 15, 2208-2216 (1996)). Further it is disclosed in Fig 4 of Nature Medicine Volume 5, Number 2, February 1999, p.223-225 that a known ROCK inhibitor which is different from hexahydro-1-(5-isoquinolinesulfonyl)-1H-1,4-diazepine hydrochloride has an inhibitory effect on the peritoneal dissemination of MM-1 cells (Rat MM-1 hepatoma cells) and on augmentation of the peritoneal invasion of MM-1 hepatoma cells *in vivo* by an osmotic pumps' implantation of the known ROCK inhibitor into the peritoneal cavity.

On the other hand hexahydro-1-(5-isoquinolinesulfonyl)-1H-1,4-diazepine hydrochloride is a commercially available medicine which is called as "Eril®Inj. (Fasudil Hydrochloride injection solution)" and has the following chemical formula.

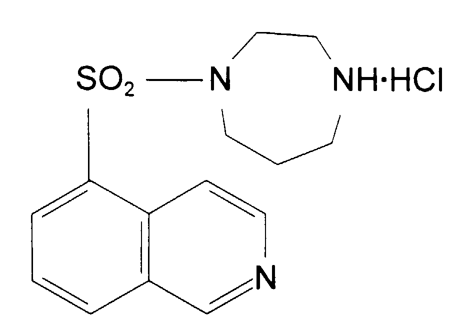

Hexahydro-1-(5-isoquinolinesulfonyl)-1H-1,4-diazepine (another chemical name is 1-(5-isoquinolinesulphonyl)homopiperazine: hereinafter called as "Fasudil") and hexahydro-1-(5-isoquinolinesulfonyl)-1H-1,4-diazepine hydrochloride (hereinafter called as "Fasudil Hydrochloride") and their preparing methods are disclosed in Japanese examined publication number 5-3851, European patent publication number 187371 B1, etc.. It is disclosed in Noushinkei 45(9), 819-824 (1993) (Japan) that Fasudil Hydrochloride has (i) calcium antagonism function, (ii) inhibitiory function of protein kinase (protein phopsphotransfer enzyme) such as protein kinase A, protein kinase G, protein kinase C and Myosine light chain kinase, and (iii) vasodilation function. In addition, it is disclosed in Japanese unexamined publication number 10-87491 that Isoquinoline derivatives such as Fasudil are a nuclear factor kappa B (NF-kB) protein kinase (protein phopsphotransfer enzyme) inhibitor and is useful for an agent for controlling cancer invasion. It is also known that HA1077 [Fasudil Hydrochloride; It is described in Br. J. Pharmacol. 98, 1091-1100 (1989) that HA 1077 is 1-(5-isoquinolinesulphonyl)-homopiperazine HCI] is an inhibitor of ROCK by the data in Table 1 of Nature Vol. 389/ 30 October 1997, p. 991.

However, none of the protein kinase A, protein kinase G, protein kinase C, Myosine light chain kinase and NF-kB protein kinase (protein phopsphotransfer enzyme), is a downstream kinase of Rho, and the inhibition potency to these kinases is much weaker than to Rho downstream kinases [According to Ki value of protein kinase A, protein kinase G, protein kinase C and myosine light chain kinase described in Noushinkei 45(9), 819-824 (1993) (Japan); Ki value of p160ROCK described in Table 1 of Nature vol. 389/ 30 October 1997, p.991; and the inhibitory effect of NF-kB protein kinase described in Table 1 of Japanese unexamined publication number 10-87491]. The above mentioned Japanese unexamined publication number 10-87491 discloses that the effect of controlling cancer invasion is recognized by controlling activity of NF-kB (Cancer Res. 55: 4162-67 (1995)). However, the authors of the Cancer Res. found N-acetyl N-cysteine, aspirin or pentoxifylline inhibits cancer cells invasion in their model, but they did not show this invasion is mediated via inhibition of NF-kB function. Furthermore, there is no evidence widely accepted that NF-kB inhibition leads to inhibition of cancer invasion.

In addition, the mechanism of relationship between cancer invasion and the downstream kinases of Rho such as PKN and ROCK have not been completely solved yet.

Further, none of the references disclose Fasudil or Fasudil hydrochroride is a PKN inhibitor or a dual inhibitor of PKN and ROCK and none of the references disclose Fasudil or Fasudil hydrochroride controls "Carcinomatous peritonitis" derived from intraabdominal tumor and/or "Carcinomatous pleuritis" derived from intrathoracic tumor. Especially, none of the references disclose a PKN inhibitor, a ROCK inhibitor or a dual inhibitor of PKN and ROCK controls the peritoneal dissemination of tumor and on augmentation of the peritoneal invasion of tumor *in vivo* by oral administration and parenteral administration (other than implantation) such as intravenous administration.

### SUMMARY OF THE INVENTION

The present invention is based on PKN inhibitory effect by Fasudil/ Fasudil Hydrochloride and dual inhibition of PKN and ROCK by Fasudil/ Fasudil Hydrochloride.

Due to either the PKN inhibition or the dual inhibition of PKN and ROCK, Fasudil/ Fasudil Hydrochloride has the effect of controlling cancer invasion and the effect of controlling carcinomatous peritonitis derived from intraabdominal tumor. Fasudil/ Fasudil Hydrochloride may be useful for controlling carcinomatous pleuritis derived from intrathoracic tumor because the pathogenesis mechanism of carcinomatous pleuritis derived from intrathoracic tumor is similar to that of carcinomatous peritonitis derived from intraabdominal tumor.

### DETAILED DESCRIPTION

The present invention is related to (i) a protein kinase N inhibitor comprising hexahydro-1-(5-isoquinolinesulfonyl)-1H-1,4-diazepine or its salts, (ii) a dual inhibitor of protein kinase N (hereinafter called as "PKN") and a Rho-associated, coiled-coil containing protein kinase (p160ROCK) (hereinafter called as "ROCK") comprising hexahydro-1-(5-isoquinolinesulfonyl)-1H-1,4-diazepine or its salts and (iii) a pharmaceutical composition for controlling carcinomatous peritonitis derived from intraabdominal tumor comprising hexahydro-1-(5-isoquinolinesulfonyl)-1H-1,4-diazepine or its salts. Fasudil/ Fasudil Hydrochloride may be useful for controlling carcinomatous pleuritis derived from intrathoracic tumor because the pathogenesis mechanism of carcinomatous pleuritis derived from intrathoracic tumor is similar to that of carcinomatous peritonitis derived from intraabdominal tumor. Thus (iv) a pharmaceutical composition for controlling carcinomatous pleuritis derived from intrathoracic tumor comprising hexahydro-1-(5-isoquinolinesulfonyl)-1H-1,4-diazepine or its salts is also included in the present invention. Further, (v) a pharmaceutical composition for controlling cancer invasion comprising hexahydro-1-(5-isoquinolinesulfonyl)-1H-1,4-diazepine or its salts is also included in the present invention.

The intraabdominal tumor includes gastric cancer, hepatic cancer, pancreatic cancer, colorectum cancer and ovarian cancer. The intrathoracic tumor includes lung cancer. The term "controlling" used herein includes the meaning of preventing and treating

Hexahydro-1-(5-isoquinolinesulfonyl)-1H-1,4-diazepine hydrochloride [another chemical name is 1-(5-isoquinolinesulphonyl)homopiperazine hydrochloride] is commercially available medicine which is called as "Eril®Inj. (Fasudil Hydrochloride injection solution)" and has the following chemical formula.

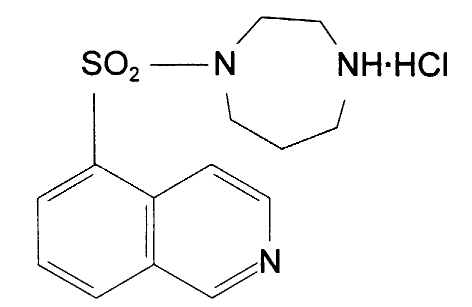

Hexahydro-1-(5-isoquinolinesulfonyl)-1 H-1,4-diazepine (another chemical name is 1-(5-isoquinolinesulphonyl)homopiperazine: hereinafter called as "Fasudil") and hexahydro-1-(5-isoquinolinesulfonyl)-1H-1,4-diazepine hydrochloride (hereinafter called as "Fasudil Hydrochloride") and their preparing methods are disclosed in Japanese examined publication number 5-3851, European patent publication number 187371 B1, etc.. It is described in Drugs in Japan Ethical Drugs (published by Yakugyo Jiho Co., Ltd. Tokyo Japan) and the package insert of Eril®Inj. that LD 50 (mg/Kg) of Fasudil Hydrochloride is 67.6 (male mouse, i.v.) and 72.5 (female mouse, i.v.). The salt of hexahydro-1-(5-isoquinolinesulfonyl)-1H-1,4-diazepine is a pharmaceutically acceptable acid addition salt. The acid addition salt includes salts with inorganic acids such as hydrochloric acid, hydrobromic acid, phosphoric acid and sulfuric acid, and salts with organic acids, such as acetic acid, citric acid, tartaric acid, lactic acid, succinic acid, fumaric acid, maleic acid and methanesulfonic acid. Among them, the hydrochloride (a salt with hydrochloric acid) salt is preferable. The hexahydro-1-(5-isoquinolinesulfonyl)-1H-1,4-diazepine hydrochloride can be hexahydro-1-(5-isoquinolinesulfonyl)-1H-1,4-diazepine hydrochloride trihydrates (Japanese unexamined Patent publication number 9-12573) or from 1/2 to 3 hydrates of hexahydro-1-(5-isoquinolinesulfonyl)-1H-1,4-diazepine hydrochloride (PCT International Publication Number W097/02260). The trihydrates and the 1/2 to 3 hydrates are also included in the hexahydro-1-(5-isoquinolinesulfonyl)-1H-1,4-diazepine hydrochloride which is used in the present invention: (i) the pharmaceutical composition for controlling cancer invasion comprising hexahydro-1-(5-isoquinolinesulfonyl)-1H-1,4-diazepine or its salts of the present invention, (ii) the pharmaceutical composition for controlling carcinomatous peritonitis derived from intraabdominal tumor comprising hexahydro-1-(5-isoquinolinesulfonyl)-1H-1,4-diazepine or its salts of the present invention and (iii) the pharmaceutical composition for controlling carcinomatous pleuritis derived from intrathoracic tumor comprising hexahydro-1-(5-isoquinolinesulfonyl)-1H-1,4-diazepine or its salts of the present invention, further comprises at least one pharmaceutically acceptable carrier or diluent. Examples of carriers which may be employed include binders such as gelatin, hydroxypropyl cellulose, starch, carboxymethyl cellulose, gum arabic, glucose, tragacanth, sucrose and sodium alginate; vehicles such as crystalline cellulose, glucose, starch, lactose and sucrose; lubricants such as calcium stearate, magnesium stearate, stearic acid and refined talc; disintegrators such as calcium carbonate, carboxymethyl cellulose and starch; additives such as glycerin, lecithin and soybean oil; and the like. Examples of diluents include distilled water, ethanol, physiological saline solution, vegetable oil, Ringer's solution and mixtures thereof. In case where Fasudil or Fasudil Hydrochloride is formulated into an inhalant, polychloromonofluoromethane or the like may be used as a solvent. Further, Fasudil or Fasudil Hydrochloride may be used in combination with other drugs, depending on the symptoms of a patient. When Fasudil or Fasudil Hydrochloride is administered to humans, it may be orally administered in the form of tablet, powder, granule, capsule, suger-coated tablet, suspension and syrup, or parenterally administered in the form of solution or suspension for injection, cream and spray. Said injection includes intravenous injection, hypodermic injection, intramuscular injection and intramedullary injection. Fasudil or Fasudil Hydrochloride can be directely implanted into the diseased part, however oral administration and parenteral administration (other than implantation) such as intravenous administration are preferred. When the formulations of Fasudil or Fasudil Hydrochloride are tablet, capsule, powder or granule by oral administration, 0.01-100 wt. %, preferably 1-40 wt. % of active ingredient (Fasudil or Fasudil Hydrochloride) should be included in the formulation. When the formulations of Fasudil or Fasudil Hydrochloride are suspension or syrup by oral administration, preferably 0.01-20 wt. % of active ingredient (Fasudil or Fasudil Hydrochloride) should be included in the formulation. When the formulations of Fasudil or Fasudil Hydrochloride are solution or suspension for injection, 0.001-20 wt.%, preferably 0.01-10 wt.% of active ingredient (Fasudil or Fasudil Hydrochloride) should be included in the formulation. The dose is varied depending on the age, weight, condition, etc. of the patient. However, the dose may generally be 10-10000 mg per day for an adult. The daily dose may be administered at one time, or it may also be derived into 2 or 3 portions and these portions are administered at intervals. The administration is generally continued for a period of from several days to 2 months. The daily dose and the administration period are varied to some extent depending on the condition of the patient.

### EXAMPLES

Hereinbelow, the present invention will be described in detail with reference to the following Examples but they should not be construed to be limiting the scope of the present invention.

### EXAMPLE 1

### Measurement of inhibition activity of protein kinase N (PKN) by Fasudil Hydrochloride

The PKN reaction was carried out in 50 µl reaction buffer (20mM Tris HCI pH 7.5, 8mM MgCl₂) containing (i) 40µM PKCα peptide (RFARKGSLRQKNVHEVK) as a substrate of PKN, (ii) 1 µM GTPγS form GST-RhoA (prepared by the method of Method in ENZYMOLOGY Vol. 256 Small GTPases and Their Regulators Part B Rho Family P.3-10), (iii) 50µM, 20µM, 10µM and 5µM (as the final concentration of the reaction mixture) of ATP and 0.5µl of [γ-³²P] ATP (1.11 TBg/mmol, 74MBg/ml; supplied by NEN^{™} Life Science Product, Inc.), and (iv) a purified human-recombinant protein kinase N (prepared by using a Baculovirus expression system), in the presence of or in the absence of 1 µM of hexahydro-1-(5-isoquinolinesulfonyl)-1H-1,4-diazepine hydrochloride (Fasudil Hydrochloride). 30µl of the reaction mixture was spotted onto Whatman paper P81 (supplied by Whatman International Ltd.) after the incubation for 10 minutes at 30°C. After the spotting, the spotted Whatman paper was washed with 75mM phosphoric acid 5 times. Then incorporation of ³²P into the PKCα peptide on the washed paper was assessed by scintillation counting. Based on the assessment by scintillation counting, Km value was measured by Lineweaver-Burk plot (method), then K, value was calculated.

The calculated Ki value: Kᵢ=922nM,

According to the value "Kᵢ=922nM" it can be understood that the PKN inhibitory activity of Fasudil Hydrochloride is ten times higher than the inhibitory activity of protein kinase C by Fasudil Hydrochloride (HA 1077) described in the Table 1 of Nature vol. 389, p. 991/30 October 1997 based on the Kᵢ values.

### ROCK Inhibition by Fasudil Hydrochloride

According to Table 1 of Nature vol. 389/ 30 October 1997, p.991, it can be understood that the ROCK inhibitory activity of Fasudil Hydrochloride is 28 times higher than the inhibitory activity of protein kinase C by Fasudil Hydrochloride (HA 1077) described in the Table 1 of Nature vol. 389, p. 991/30 October 1997 based on the Ki values.

### Effect of Fasudil Hydrochloride on the Peritoneal Invasion of Rat MM-1 Ascites Hepatoma cells bearing V14 Rho A (effect on carcinomatous peritonitis derived from intraabdominal tumor)

MMI ascites hepatoma cells (2x10⁷) stably expressing Val 14-RhoA were injected into the peritoneal cavities of male Donryu rats (body weight 100g) (to obtain a model of rat having MM-1 ascites Hepatoma cells bearing V14 Rho A). Fasudil was intravenously delivered at the indicated dose (0, 0.1 mg/Kg, 1 mg/Kg, and 10 mg/Kg) 3 times per day. After 11 days, the animals were sacrificed and (i) tumor nodules and tumor cell dissemination on peritoneum were assessed (This test was conducted by the method which is similar to one described in Nature Medicine Volume 5, Number 2, February 1999, p.223-225 and was conducted with 5 rats at each dose.). In addition, hyperemia of the surface in the peritoneum was also observed. The results are shown in Table 1.

**Table 1**

| | | | | |
|---|---|---|---|---|
| Dose of Fasudil Hydrochloride (mg/Kg) | 0 | 0.1 | 1 | 10 |
| Total number of tumor nodules on peritoneum | 20 | 22 | 11 | 0 |
| Hyperemia of the surface in the peritoneum | +++ | + | ± | ± |

In Table 1, the symbols +++,++, +, and ± mean as follows:
+++: more than 75% of surface in the peritoneum shows hyperemia
++: 50-75% of surface in the peritoneum shows hyperemia
+: 25-50% of surface in the peritoneum shows hyperemia
±: less than 25% of surface in the peritoneum shows hyperemia

The result of hyperemia of the surface in the peritoneum shows that Fasudil Hydrochloride controls carcinomatous peritonitis derived from intraabdominal tumor.

## Claims

1. The use of hexahydro-1-(5-isoquinolinesulfonyl)-1H-1,4-diazepine or its salts for the manufacture of a medicament which is a protein kinase N inhibitor.

2. The use of hexahydro-1-(5-isoquinolinesulfonyl)-1H-1,4-diazepine hydrochloride for the manufacture of a medicament which is a protein kinase N inhibitor.

3. The use of hexahydro-1-(5-isoquinolinesulfonyl)-1H-1,4-diazepine or its salts for the manufacture of a medicament which is a dual inhibitor of protein kinase N and a Rho-associated, coiled-coil containing protein kinase (p160ROCK).

4. The use of hexahydro-1-(5-isoquinolinesulfonyl)-1H-1,4-diazepine hydrochloride for the manufacture of a medicament which is a dual inhibitor of protein kinase N and a Rho-associated, coiled-coil containing protein kinase (p160ROCK).

5. A protein kinase N inhibitor comprising hexahydro-1-(5-isoquinolinesulfonyl)-1H-1,4-diazepine or its salts.

6. The protein kinase N inhibitor according to claim 5 wherein said hexahydro-1-(5-isoquinolinesulfonyl)-1H-1,4-diazepine or its salts is hexahydro-1-(5-isoquinolinesulfonyl)-1H-1,4-diazepine hydrochloride.

7. A dual inhibitor of protein kinase N and a Rho-associated, coiled-coil containing protein kinase (p160ROCK) comprising hexahydro-1-(5-isoquinolinesulfonyl)-1H-1,4-diazepine or its salts.

8. The dual inhibitor of protein kinase N and a Rho-associated, coiled-coil containing protein kinase (p160ROCK) according to claim 7 wherein said hexahydro-1-(5-isoquinolinesulfonyl)-1H-1,4-diazepine or its salts is hexahydro-1-(5-isoquinolinesulfonyl)-1H-1,4-diazepine hydrochloride.

9. A pharmaceutical composition for controlling carcinomatous peritonitis derived from intraabdominal tumor comprising hexahydro-1-(5-isoquinolinesulfonyl)-1H-1,4-diazepine or its salts.

10. A pharmaceutical composition for controlling carcinomatous peritonitis derived from intraabdominal tumor according to claim 9 wherein said hexahydro-1-(5-isoquinolinesulfonyl)-1H-1,4-diazepine or its salts is hexahydro-1-(5-isoquinolinesulfonyl)-1 H-1,4-diazepine hydrochloride.

11. A pharmaceutical composition according to claim 10 wherein the pharmaceutical composition further comprises at least one pharmaceutically acceptable carrier or diluent and is used for oral administration and parenteral administration other than implantation.

12. A pharmaceutical composition according to claim 11 wherein the pharmaceutical composition is used for intravenous administration and comprises at least one pharmaceutically acceptable diluent.

13. A pharmaceutical composition for controlling carcinomatous pleuritis derived from intrathoracic tumor comprising hexahydro-1-(5-isoquinolinesulfonyl)-1H-1,4-diazepine or its salts.

14. A pharmaceutical composition for controlling carcinomatous pleuritis derived from intrathoracic tumor according to claim 13 wherein said hexahydro-1-(5-isoquinolinesulfonyl)-1 H-1,4-diazepine or its salts is hexahydro-1 -(5-isoquinolinesulfonyl)-1 H-1,4-diazepine hydrochloride.

15. A pharmaceutical composition according to claim 14 wherein the pharmaceutical composition further comprises at least one pharmaceutically acceptable carrier or diluent and is used for oral administration and parenteral administration other than implantation.

16. A pharmaceutical composition for controlling cancer invasion comprising hexahydro-1-(5-isoquinolinesulfonyl)-1H-1,4-diazepine or its salts.

17. A pharmaceutical composition for controlling cancer invasion according to claim 12 wherein said hexahydro-1-(5-isoquinolinesulfonyl)-1H-1,4-diazepine or its salts is hexahydro-1-(5-isoquinolinesulfonyl)-1H-1,4-diazepine hydrochloride.

18. A pharmaceutical composition according to claim 17 wherein the pharmaceutical composition further comprises at least one pharmaceutically acceptable carrier or diluent and is used for oral administration and parenteral administration other than implantation.

19. A pharmaceutical composition according to claim 18 wherein the pharmaceutical composition is used for intravenous administration and comprises at least one pharmaceutically acceptable diluent.
